# FASCICULE DE BREVET EUROPEEN

(11) **EP 2 226 252 B1**
(45) Date de publication et mention de la délivrance du brevet: **05.09.2012**
(21) Numéro de dépôt: 09178560.0
(22) Date de dépôt: 09.12.2009
(51) Int. Cl.: B65B 7/28, B65B 31/02, B65B 55/02, A23L 3/02, A23L 3/3418, B65B 43/50, B65B 55/10

(54) **Procédé et installation pour le conditionnement sous vide en continu de produits alimentaires**
Verfahren und Anlage zur fortlaufenden Vakuumverpackung von Lebensmitteln
Method and installation for continuous vacuum packing of food products

(30) Priorité: 02.03.2009 FR 0900966
(43) Date de publication de la demande: 08.09.2010
(73) Titulaire: Sodetech SARL, 33260 La Teste de Buch (FR)
(72) Inventeur: Le Goff, épouse Larroche, Suzanne, 47300 Pujols (FR); Larroche, Brigitte, 75020 Paris (FR)
(74) Mandataire: Ravina, Bernard

(56) Documents cités:
- FR-A- 2 385 607
- FR-A- 2 686 059
- FR-A- 2 829 106
- US-A- 1 931 911

## Description

La présente invention concerne un procédé et une installation pour le conditionnement sous vide en continu de produits alimentaires pouvant être pasteurisés ou stérilisés dans un récipient en verre, en métal ou en plastique dont la fermeture s'effectue par un couvercle exempt de moyens d'accrochage et pourvu d'un joint élastique maintenu par un vide poussé selon les préambules des revendications 1 et 12.

Le brevet US n° 1 931 911 décrit une méthode de conditionnement sous vide de produits alimentaires contenus dans un récipient et destiné à être fermé par un couvercle dépourvu de moyens d'accrochage.

Le récipient et le couvercle sont placés dans une cloche autoclave dans laquelle est réalisée une injection vapeur puis un vide, le couvercle étant plaqué sur le récipient et maintenu par le vide.

Cette méthode est inadaptée à une production en série.

Le brevet français REY n° 2385607 décrit un procédé pour le conditionnement et la conservation de matières périssables et l'emballage pour sa mise en oeuvre.

Ce brevet décrit un procédé dans lequel est injectée de la vapeur entre le couvercle et le récipient puis à introduire le récipient dans un autoclave en créant une surpression dans celui-ci, cette surpression étant maintenue pendant toute la durée de pasteurisation ou de stérilisation puis à refroidir progressivement le récipient et à rétablir la pression normale.

La surpression à l'intérieur de l'autoclave est mentionnée comme un moyen essentiel de l'invention obtenu par injection d'air comprimé dans l'autoclave pour maintenir la capsule ou couvercle sur le récipient.

En fait, il apparaît qu'il est impossible de pasteuriser ou de stériliser en injectant ou introduisant en même temps de l'air comprimé et de la vapeur dans un autoclave ; le mélange d'air et de vapeur est hétérogène, et la température dans l'autoclave est de ce fait également hétérogène. Seuls des autoclaves spéciaux qui sont équipés d'énormes ventilateurs à l'intérieur de l'autoclave peuvent utiliser ce mélange en le rendant homogène par très fort brassage du ventilateur intérieur de l'autoclave.

De même, le temps de stérilisation nécessaire pour obtenir la sécurité de conservation amène obligatoirement à une cuisson excessive du produit. II est impensable de laisser refroidir le produit dans l'autoclave sans intervenir par un refroidissement rapide effectué par un circuit d'eau froide dans l'autoclave. Le refroidissement très lent que préconise le texte amènerait à une dégradation de tout produit par une caramélisation des sucres ou une dégradation des goûts et de la couleur par l'effet de Maillard ou les deux effets associés.

Le brevet d'invention CATRAIN n° 2686059 a recours aux mêmes procédé et moyen que le brevet REY et est sujet aux mêmes impossibilités et inconvénients.

L'invention vise un procédé et une installation qui permet de réaliser en continu, et sans utilisation obligatoire d'un autoclave, un conditionnement de produits alimentaires sous vide pouvant être pasteurisé ou stérilisé dans un récipient rigide en verre ou en métal dont la fermeture s'effectue par un couvercle exempt de moyens d'accrochage au récipient et pourvu d'un joint élastique, le couvercle étant maintenu par un vide poussé et dont l'ouverture s'effectuant par un système de casse vide, ce qui évite les inconvénients de l'art antérieur.
Notamment, l'invention vise à éviter la surcuisson du produit, sa caramélisation et à ne pas modifier le goût et la couleur du produit.

A cet effet, l'invention est définie dans les revendications 1 et 12. Elle se caractérise essentiellement en ce que le couvercle est maintenu à distance du récipient préalablement rempli, que de la vapeur surchauffée est injectée à une température, un débit et en un temps qui permette la décontamination du couvercle et de l'espace de tête du récipient en chassant également l'air et les incondensables, notamment l'oxygène, ce qui réalise le vide, que le récipient est pressé contre le couvercle ou inversement et qu'un jet d'eau froide est envoyé sur le couvercle, ce qui provoque un refroidissement rapide du couvercle, la condensation immédiate de la phase vapeur dans l'espace de tête du récipient et la mise sous vide profond.

Avantageusement, le couvercle est maintenu à distance, 5 à 10 millimètres, du récipient préalablement rempli. La vapeur injectée entre le couvercle et le récipient est surchauffée à 120°-135° pour avoir un effet décontaminant et également réduire le volume de condensat dans l'emballage.

D'autres caractéristiques et avantages de l'invention apparaîtront à la lecture de la description ci-après du procédé et de l'installation de mise en oeuvre illustrée à titre d'exemple non limitatif par les dessins joints dans lesquels :
- la figure 1 est une vue schématique de l'installation mettant en oeuvre le procédé selon l'invention;
- la figure 2 est une vue en coupe du dispositif de mise en place des couvercles sur le récipient;
- la figure 3 est une vue en coupe du mandrin mobile de mise en place du couvercle organisé pour effectuer le refroidissement rapide selon l'invention;
- la figure 4 est une vue de dessous du mandrin avec les aimants de maintien du couvercle;
- les figures 5A-5B montrent un couvercle avec un casse vide par le décollage d'une languette thermo scellée;
- les figures 6A-6B illustrent un couvercle avec un casse vide du type à anneau à percussion;
- la figure 7 représente un type de récipient.

L'invention concerne un procédé et ses moyens de mise en oeuvre (machine, installation, etc...) pour le conditionnement en continu de produits alimentaires sous vide, avant ou après pasteurisation ou stérilisation, dans un récipient rigide dont la fermeture se fait avec un couvercle rigide, bloqué en position par le différentiel de pression entre l'intérieur du récipient et l'extérieur.

Le couvercle rigide est dépourvu de moyens d'accrochage mécanique au récipient, l'ouverture de celui-ci s'effectue de manière facile par un système de casse vide.

Le procédé selon l'invention peut également être utilisé pour conditionner un produit super propre destiné à une distribution par la chaîne du froid sans pasteurisation ni stérilisation.

Les emballages ou récipients utilisés dans la mise en oeuvre du procédé doivent autant que possible répondre aux spécifications suivantes :
- soit être des pots en verre dont le bord est éventuellement arrondi et éventuellement muni de rainures dans l'épaisseur pour permettre l'adhésion facile du joint du couvercle;
- soit être des boîtes en métal dont le bord est roulé ou replié, créant un appui et une surface sur laquelle le joint du couvercle va adhérer;
- soit être des pots en matière synthétique dite plastique à condition qu'ils présentent une structure offrant une bonne résistance à la différence de pression entre l'extérieur et l'intérieur du récipient.

Dans tous les cas, il est préférable que les récipients, pots ou boîte, comprennent un jonc en relief périphérique sous le niveau du couvercle pour :
- éviter que les bords de couvercle côte à côte ne se touchent lors des transferts sur ligne après fermeture;
- que le jonc en relief crée une zone de fixation pour tout sur-couvercle qui peut être en tous matériaux adaptés, matière plastique, métaux, carton, qui permette ainsi une consommation partielle des produits et le rebouchage du récipient. Le jonc a en outre dans le cas de boite métal ou matière plastique une fonction de rigidification du col de la boite.

Le couvercle métallique utilisé présente les caractéristiques suivantes :
- avoir un joint de composition étudié pour être ni trop rigide, ni trop souple permettant une adhésion parfaite sur le contenant durant la pression entre l'extérieur et l'intérieur du récipient;
- de préférence le couvercle doit présenter un décrochage ou rabat sur le bord externe pour s'encastrer exactement sur le rebord du récipient.

Selon l'invention, le procédé pour le conditionnement sous vide en continu de produits alimentaires pouvant être pasteurisé et/ou stérilisé (avant ou après fermeture) dans un récipient rigide, du type décrit précédemment, dont la fermeture s'effectue au moyen d'un couvercle métallique exempt de moyens ou système d'accrochage mécanique au récipient; ledit couvercle étant muni d'un joint élastique et maintenu par un vide poussé dont l'ouverture s'effectue par un système de casse vide. Une injection de vapeur surchauffée permet la décontamination du couvercle et de l'espace de tête du récipient en chassant l'air et les incondensables, notamment l'oxygène, ce qui réalise le vide que le récipient est pressé contre le couvercle ou inversement et qu'un jet d'eau froide est envoyé sur le couvercle, ce qui provoque un refroidissement rapide du couvercle, la condensation immédiate de la phase vapeur dans l'espace de tête du récipient et la mise sous vide profond.

Le produit peut être préalablement stérilisé en continu puis refroidi en continu, le produit étant emboîté à une température inférieure à celle du pasteurisé pour respecter les sucres et éviter la caramélisation et les protéines ou la couleur et éviter la réaction de Maillard.

Avantageusement, les récipients tels que décrits passent à l'entrée d'une remplisseuse dans un tunnel de préchauffage où ils sont soumis à un flux de vapeur surchauffée pour décontaminer leur surface.
Il est possible de prévoir une pré-stérilisation du récipient au moyen d'un tunnel sous rayons ultraviolets ou par une pulvérisation d'H₂O₂ qui doit précéder le flux vapeur afin de sécuriser parfaitement la décontamination.
Cette sécurisation permet d'effectuer un remplissage aseptique du récipient, le produit de remplissage pouvant dans ce cas être froid et stérile.

Dans le cas de produits devant être pasteurisés (produits dont le PH est inférieur à 4,5), les récipients sont remplis avec du produit chaud préalablement dégazé, à une température minimum permettant une autopasteurisation du récipient en contact avec le produit par exemple à la température de 90° centigrade par tout moyen de remplissage par exemple une doseuse rotative.
Dans ce cas, la température du produit permet une auto-pasteurisation du produit lui-même et des parois du récipient en contact avec le produit.

Le récipient rempli est alors transporté vers un dispositif de capsulage.
Les couvercles sont placés dans un magasin tubulaire de distribution et sont soumis à une décontamination par injection de vapeur surchauffée (125° à 130°) éventuellement précédée d'une pulvérisation d'H₂O₂.

Ils sont ensuite positionnés respectivement à un mandrin de capsulage auquel ils sont maintenus par des aimants permanents.
Les récipients sont conduits sur un support sous chaque mandrin de capsulage. Ils ont préalablement circulé dans un conduit clos ou tunnel contenant de la vapeur surchauffée de telle sorte que la plus grande partie des gaz incondensables puisse être éliminée de l'espace de tête du récipient, ce qui maintient la décontamination du col du récipient.
Les récipients se positionnent sous les couvercles à une distance de 5 à 10 millimètres, et de préférence de 5 millimètres.
De chaque côté du récipient et du couvercle superposé à distance d'environ 5 millimètres, sont disposés des injecteurs de vapeur surchauffée qui injectent ladite vapeur dans l'espace créé entre récipient et couvercle.
Cette injection de vapeur surchauffée effectue un balayage de la zone avec pour effet d'éliminer l'air résiduel contenu dans l'espace entre le couvercle et le produit dans le récipient, c'est à dire dans l'espace de tête du récipient.

La vapeur surchauffée est appliquée à une température (120-135°), un débit et en un temps qui permet de parachever l'élimination des incondensables résiduels de l'espace de tête du récipient.
Il a été constaté que plus le produit dans le récipient était froid, plus le débit de vapeur devra être élevé.

Ce balayage final a pour effet d'éliminer de l'espace de tête tous les gaz incondensables, air et oxygène et d'obtenir après refroidissement un vide profond, ce qui évite toute dégradation par oxydation sur le produit.

Le mandrin portant le couvercle est alors appuyé et pressé sur le récipient ou inversement, ce qui comprime le joint du couvercle, et un jet d'eau froide est envoyé sur le couvercle, par circulation d'eau dans le mandrin, ce qui provoque un refroidissement rapide, la condensation immédiate de la phase vapeur dans l'espace de tête et la mise sous vide profond dudit espace de tête qui maintient le couvercle en position.

La nature du joint du couvercle et la forme des bords du récipient permettent de résister aux différences de pression et d'assurer l'herméticité durant toute la durée de commercialisation du produit.
Après leur fermeture et leur refroidissement partiel dans la capsuleuse, les pots ou les boites remplis, maintenant fermés par un couvercle hermétique, avancent alors dans un tunnel de refroidissement.
En effet, le vide créé dans le pot ou la boîte provoquent l'ébullition du produit, le produit se refroidissant par la chaleur prise par l'ébullition qui provoque de la vapeur. Cette vapeur augmente à nouveau la pression dans l'espace de tête, mais se condense immédiatement par contact avec le couvercle sur lequel coule l'eau de refroidissement du tunnel. Le tout se déroule pratiquement en même temps et de façon répétitive, assurant ainsi le refroidissement très rapide du produit.

Le vide final est plus poussé que dans les produits conditionnés en pots dit «Twist off», où il reste une fraction d'air significative, contrairement au procédé décrit ici.
Les pots ou boîtes passent par un tunnel de séchage et sont prêts pour un regroupement et sur-conditionnement.

Une opération postérieure de stérilisation en autoclave peut également avoir lieu dans le cas où les produits nécessitent un tel traitement en vue d'une longue conservation à température ambiante. Dans ce cas, on veillera à maîtriser la pression à l'extérieur du récipient dans l'enceinte de stérilisation toujours supérieure à celle naturellement provoquée par le réchauffement du produit en cours de stérilisation à l'intérieur du contenant durant les phases de montée et descente en température.

La contre-pression extérieure doit être au minimum de 300 mbar supérieure à la pression dans le récipient. Le différentiel de pression entre l'intérieur du récipient et la chambre de l'autoclave ne doit pas dépasser 950 mbar.

Le produit peut être emboîté dans le contenant à une température basse, la stérilisation s'effectuant après la fermeture du contenant.
A la sortie de la machine rotative de capsulage, après le pré-refroidissement effectué sur la machine elle-même, le contenant se trouve à un différentiel de pression dépendant de la température du produit contenu; ce différentiel doit être d'un minimum de 300 mbar de vide afin d'assurer le maintien efficace du couvercle sur le corps au cours des transferts suivants.
Le contenant ainsi hermétiquement fermé, peut passer dans un appareil de stérilisation (un autoclave) muni d'un système dit de contre pression.
La contre pression doit être de 300 mbar minimum supérieure à la pression correspondante à la pression produite dans le contenant, par la température choisie pour obtenir la valeur stérilisatrice (VS) recherchée. Le refroidissement du contenant doit s'effectuer dans l'autoclave accompagnée d'une contre pression qui doit diminuer à mesure que le produit lui-même se refroidit. Le différentiel de pression entre l'intérieur du contenant et la chambre de l'autoclave ne doit pas dépasser 950 mbar.

Ce procédé permet également de traiter des produits stérilisés en méthode aseptique. Dans ce cas, l'alimentation de la remplisseuse doit être précédée d'une stérilisation en continu du produit, suivi d'un refroidissement également en continu, le corps de la remplisseuse doit être aménagé en zone d'atmosphère aseptique, la vapeur surchauffée pouvant être une solution simple et efficace.

Le produit peut être emboîté à une température inférieure à la température du pasteurisé. Température prévue pour respecter les sucres (caramélisation) ou dans l'autre cas pour respecter les protéines ou la couleur (réaction de Maillard). Le produit peut même être emboîté complètement à froid.

A la sortie de la machine capsuleuse pour la technique de pasteurisation, les pots ou les boîtes hermétiquement fermés doivent passer dans un tunnel de refroidissement.

Les avantages du procédé sont les suivants:
- la fabrication en continu permet l'obtention économique de produits de qualité supérieure,
- la durée de conservation des produits alimentaires est améliorée par l'absence d'oxygène, par un barème de pasteurisation réduit, et par le refroidissement rapide évitant toute réaction de Maillard et caramélisation,
- les produits alimentaires peuvent être conservés dans des récipients de verre, de plastique ou de métal de toute dimension,
- l'ouverture est dépourvue de risque de coupures ou de copeaux de métal tombant dans le produit,
- l'ouverture est extrêmement facile même pour les récipients de grand diamètre, contrairement aux produits conditionnés en pots dit «twist-off» où il faut limiter le niveau de vide pour que le consommateur puisse ouvrir le pot sans trop de difficulté,
- le consommateur a la preuve de la conservation et de l'intégrité du produit, par le bruit provoqué en actionnant le casse vide, le clic du couvercle qui se détend et le léger sifflement de l'air qui pénètre dans le contenant.

Les dessins annexés illustrent schématiquement et à titre d'exemple non limitatif une installation de mise en oeuvre en continu de l'invention.

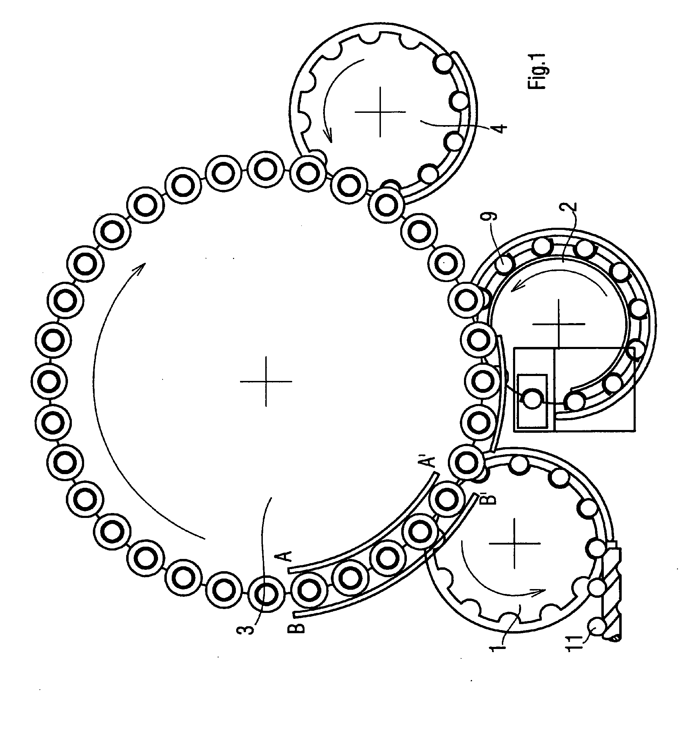

En figure 1 est représenté en plan un carrousel 1 d'alimentation des récipients préalablement remplis,

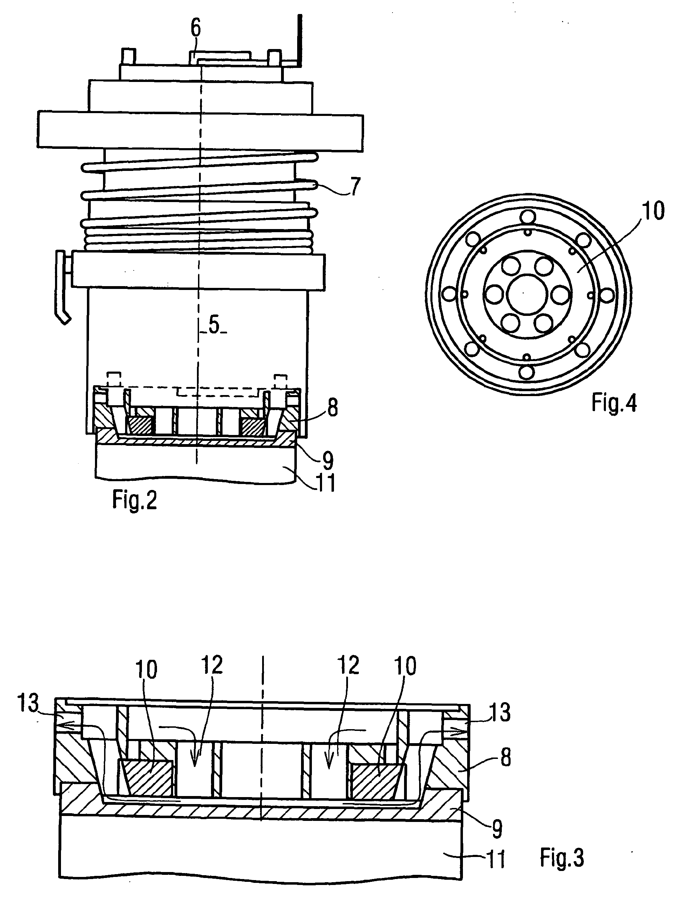

En figure 2 est représenté le carrousel d'alimentation en couvercle,

En figure 3 est représenté un carrousel circulaire dont le sens de rotation est indiqué par la flèche et qui passe successivement sur les carrousels suivants, dans la zone d'injection vapeur AA'-BB' et sur les carrousels 1 et 2 puis sur le carrousel de sortie 4.

La figure 2 représente un dispositif de distribution des couvercles dont une pluralité est portée par le carrousel 3.
Ce dispositif comprend un corps creux 5 doté à son sommet d'une valve d'alimentation en eau 6, d'un ressort 7 de compression et à sa base d'un mandrin 8 doté d'aimants permanents 10 (figure 4).

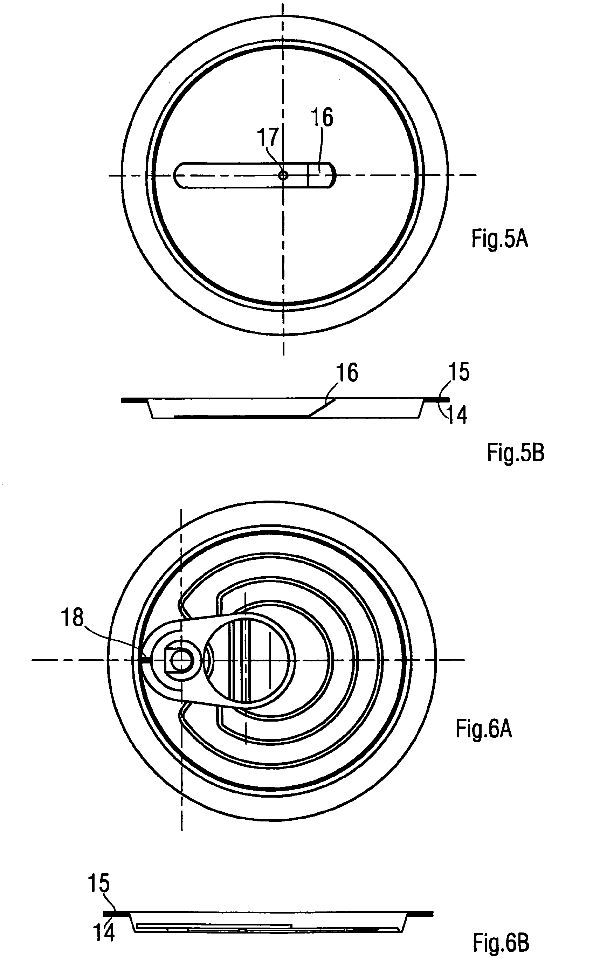

Le mandrin 8 passant sur le carrousel 2 prend le couvercle 9 et les maintient par l'action de l'aimant permanent au passage dans la zone AA'-BB' d'injection vapeur.
Après l'injection vapeur, le récipient 11 est poussé vers le mandrin 8 ou inversement sous l'action d'un piston non représenté.
Cela détermine la mise en pression du couvercle 9 et de son joint souple référencé en 14 aux figures 5 et 6 en pression sur l'orifice du récipient 11.
Conjointement, le joint 14 est compressé sur le bord du récipient.

Le mandrin 8 est alimenté en eau froide par la valve 6.
A cet effet, le mandrin est pourvu d'au moins un orifice d'entrée de l'eau 12 jusqu'au niveau du couvercle 9 et de au moins un orifice de sortie latérale de l'eau 13, ce qui effectue le refroidissement.

Aux figures 5A-5B est représenté un couvercle avec son joint 14, son rebord ou rabat 15 et un dispositif 16 de casse vide qui consiste en une languette thermocollée qui obture un orifice 17 du couvercle.

En figures 6A-6B, est représenté un couvercle muni d'un casse vide 18 du type constitué d'un anneau qui percute par basculement une zone déterminée et éventuellement prédécoupée du couvercle.

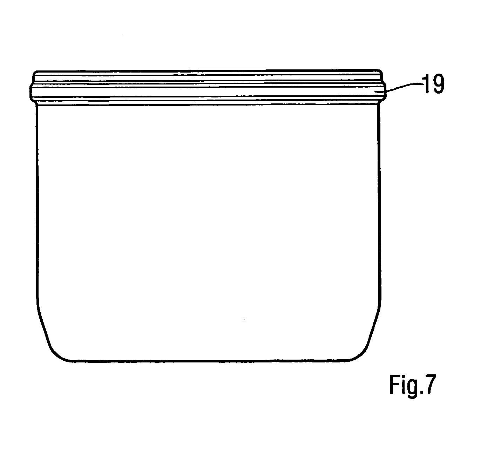

En figure 7 est représenté un récipient en verre doté de son jonc 19 périphérique sous son orifice.

## Revendications

1. Procédé pour le conditionnement en continu sous vide de produits alimentaires pouvant être pasteurisés et/ou stérilisés dans un récipient dont la fermeture s'effectue par un couvercle exempt de moyens d'accrochage et pourvu d'un joint élastique maintenu par un vide poussé ; procédé dans lequel, lors de la mise sous vide, le couvercle (9) est maintenu à distance du récipient préalablement rempli, de la vapeur surchauffée est injectée à une température, un débit et/ou un temps qui permettent la stérilisation du couvercle et de l'espace de tête du récipient en chassant l'air et les incondensables, notamment l'oxygène, le récipient étant ensuite pressé contre le couvercle ou inversement caractérisé en ce qu'un jet d'eau froide est envoyé sur le couvercle, ce qui provoque un refroidissement rapide du couvercle et la condensation immédiate de la phase vapeur dans l'espace de tête du récipient, ce qui permet la mise sous vide profond, l'ouverture s'effectuant par un système de casse-vide.

2. Procédé selon la revendication 1 **caractérisé en ce que** le couvercle (9) est maintenu à une distance entre 5 à 10 millimètres du récipient préalablement rempli. à

3. Procédé selon la revendication 1 **caractérisé en ce que** préalablement à l'injection vapeur surchauffée et à la pose du couvercle, les récipients avant remplissage sont soumis à un flux de vapeur surchauffée pour les réchauffer et décontaminer leur surface.

4. Procédé selon les revendications 1 et 2 **caractérisé en ce que** préalablement à l'injection vapeur et à la pose du couvercle (9), les récipients avant remplissage sont soumis à une préstérilisation par rayons ultraviolets.

5. Procédé selon les revendications 1 et 2 **caractérisé en ce que** préalablement à l'injection vapeur et à la pose du couvercle (9), les récipients avant remplissage sont soumis à une préstérilisation par pulvérisation d'H₂O₂.

6. Procédé selon l'une quelconque des revendications précédentes **caractérisé en ce que** la vapeur injectée entre le couvercle (9) et l'espace de tête du récipient est surchauffée à 120°-135°.

7. Procédé selon l'une quelconque des revendications 1 à 6 **caractérisé en ce que** les récipients sont remplis avec du produit chaud préalablement dégazé à une température minimum permettant une autopasteurisation du récipient en contact avec le produit.

8. Procédé selon la revendication 1 et l'une quelconque des revendications 2 à 7 **caractérisé en ce que** les récipients remplis et fermés subissent une stérilisation en autoclave, la pression à l'extérieur du récipient étant toujours supérieure à celle à l'intérieur du récipient durant les phases de montée et descente en température.

9. Procédé selon la revendication 8 **caractérisé en ce que** la contrepression est minimum de 300 mbar supérieure à la pression dans le récipient.

10. Procédé selon les revendications 8 et 9 **caractérisé en ce que** le différentiel de pression entre l'intérieur du récipient et la chambre de l'autoclave ne dépasse pas 950 mbar.

11. Procédé selon la revendication 1 **caractérisé en ce que** le produit est préalablement stérilisé en continu puis refroidi en continu, le produit étant emboîté à une température inférieure à celle du pasteurisé pour respecter les sucres et éviter la caramélisation et les protéines ou la couleur et éviter la réaction de Maillard.

12. Installation pour la mise en oeuvre du procédé selon l'une des revendications précédentes dans laquelle le conditionnement en continu sous vide de produits alimentaires pouvant être pasteurisés et/ou stérilisés dans un récipient dont la fermeture s'effectue par un couvercle exempt de moyens d'accrochage et pourvu d'un joint élastique, maintenu par un vide poussé, ladite installation comportant :
- un premier carrousel (1) d'alimentation en récipients ;
- un deuxième carrousel (2) d'alimentation en couvercles ;
- un troisième carrousel (3) doté de mandrins (8) de prise des couvercles (9) grâce à des aimants (10) sur le carrousel (2) ;
**caractérisée par** :
une zone (AA' et BB') du carrousel (3) d'injection vapeur dans l'espace créé entre le récipient et le couvercle,
- un mandrin (8) porté par un corps creux (5) surmonté d'un ressort de compression (7) et d'une vanne (6) d'alimentation en eau de refroidissement qui s'ouvre par compression du ressort, l'eau de refroidissement étant déversée sur le couvercle (9) provoquant la condensation de la vapeur dans l'espace de tête et le vide recherchépar poussée du récipient (11) vers le mandrin (8) ou inversement au moyen d'un piston.

13. Installation selon la revendication précédente **caractérisée en ce que** le mandrin (8) est doté d'un circuit d'eau passant à travers le corps creux (5) sur le couvercle (9) par au moins une entrée (12) et au moins une sortie latérale (13).

## Claims

1. Process for the continuous vacuum prepacking of food products that can be pasteurized and/or sterilized in a container that is closed by a cover that has no means of hooking and is equipped with an elastic seal that is maintained by a forced vacuum; process in which, during the placing under vacuum, the cover (9) is kept apart from the previously filled container, superheated vapor is injected at a temperature, a flow rate and/or a time that allow the sterilization of the cover and the head space of the container by expelling the air and the incondensable products, in particular oxygen; the container is then pressed against the cover or vice versa, **characterized in that** a jet of cold water is sent onto the cover, which causes rapid cooling of the cover and the immediate condensation of the vapor phase in the head space of the container, which allows the placing under deep vacuum, the opening is made by means of a vacuum breaker.

2. Process according to claim 1, wherein the cover (9) is kept at a distance, between 5 to 10 millimeters, from the previously filled container.

3. Process according to claim 1, wherein prior to the superheated vapor injection and to the placing of the cover, the containers before filling have undergone a flow of superheated vapor to heat them and to decontaminate their surfaces.

4. Process according to claims 1 and 2, wherein prior to the vapor injection and the placing of the cover (9), the containers before filling are subjected to a presterilization by ultraviolet rays.

5. Process according to claims 1 and 2, wherein prior to the vapor injection and to the placing of the cover (9), the containers, before filling, are subjected to a presterilization by spraying H₂O₂.

6. Process according to any of the preceding claims, wherein the vapor that is injected between the cover (9) and the head space of the container is superheated to 120° - 135°.

7. Process according to any of claims 1 to 6, wherein the containers are filled with the hot product that was previously degassed at a minimum temperature allowing a self-pasteurization of the container in contact with the product.

8. Process according to claim 1 and any of claims 2 to 7, wherein the containers that are filled and closed are subjected to sterilization in an autoclave, whereby the pressure outside of the container is always higher than that inside the container during the temperature rise and fall phases.

9. Process according to claim 8, wherein the counter-pressure is at least 300 mbar higher than the pressure in the container.

10. Process according to claims 8 and 9, wherein the pressure differential between the inside of the container and the chamber of the autoclave does not exceed 950 mbar.

11. Process according to claim 1, wherein the product is previously sterilized continuously and then cooled continuously, whereby the product is encased at a temperature that is lower than that of the pasteurized product in order to preserve the sugars and to prevent the caramelization and the proteins or the color and to prevent the Maillard reaction.

12. Installation for the implementation of the process according to one of the preceding claims wherein the continuous vacuum packaging of food products that can be pasteurized and/or sterilized in a container that is closed by means of a cover that has no means of hooking, and is equipped with an elastic seal and maintained by a forced vacuum, said installation comprising:
- A first container supply carrousel (1);
- A second cover supply carrousel (2);
- A third carrousel (3) that is equipped with mandrels (8) for gripping covers (9) using magnets (10) on the carrousel (2);
**characterized by**:
- A zone (AA' and BB') of the carrousel (3), for vapor injection into the space that is created between the container and the cover;
- a mandrel (8) is carried by a hollow body (5) on top of which is a compression spring (7) and a water supply valve (6) that opens by compression of the spring, whereby the cooling water is poured onto the cover (9) which causes the condensation of the vapor in the head space and the desired vacuum, by pushing the container (11) toward the mandrel (8) or vice versa by means of a piston.

13. Installation according to the preceding claim, wherein the mandrel (8) is equipped with a water circuit that passes through the hollow body (5) on the cover (9) through at least one inlet (12) and at least one lateral outlet (13).

## Patentansprüche

1. Verfahren zum kontinuierlichen Vakuumverpacken von Lebensmitteln, die in einem Behälter pasteurisiert und/oder sterilisiert werden können, welcher mit einem Deckel verschlossen wird, der keinerlei Befestigungsmittel aufweist und mit einer elastischen Dichtung versehen ist, die durch ein Hochvakuum an Ort und Stelle gehalten wird; wobei in dem Verfahren, beim Anlegen des Vakuums, der Deckel (9) von dem zuvor befüllten Behälter beabstandet gehalten wird, überhitzter Dampf mit einer Temperatur, einer Flussrate und/oder Zeitdauer eingeleitet wird, welche eine Sterilisierung des Deckels und des Kopfraums des Behälters ermöglichen, wobei Luft und nicht kondensierbare Bestandteile, insbesondere Sauerstoff, ausgetrieben werden, wobei der Behälter anschließend gegen den Deckel gepresst wird oder umgekehrt, **dadurch gekennzeichnet, dass** ein kalter Wasserstrahl auf den Deckel gelenkt wird, was ein rasches Abkühlen des Deckels und die sofortige Kondensation der Dampfphase im Kopfraum des Behälters bewirkt, sodass ein starkes Vakuum hergestellt werden kann, wobei das Öffnen mittels eines Systems zur Aufhebung des Vakuums erfolgt.

2. Verfahren nach Anspruch 1, dadurch gekenntzeichnet, dass der Deckel (9) in einem Abstand zwischen 5 und 10 Millimetern vom zuvor befüllten Behälter gehalten wird.

3. Verfahren nach Anspruch 1, **dadurch gekennzeichnet, dass** vor dem Einleiten überhitzten Dampfes und dem Aufsetzen des Deckels die noch unbefüllten Behälter einem Strom überhitzten Dampfes aufgesetzt werden, um sie zu erhitzen und ihre Oberfläche zu entkeimen.

4. Verfahren nach den Ansprüchen 1 und 2, **dadurch gekennzeichnet, dass** vor der Dampfeinleitung und dem Aufsetzen des Deckels (9) die noch unbefüllten Behälter einer Vorsterilisierung durch ultraviolette Strahlung ausgesetzt werden.

5. Verfahren nach den Ansprüchen 1 und 2, **dadurch gekennzeichnet, dass** vor der Dampfeinleitung und dem Aufsetzen des Deckels (9) die noch unbefüllten Behälter einer Vorsterilisierung durch Versprühen von H₂O₂ ausgesetzt werden.

6. Verfahren nach einem beliebigen der vorhergehenden Ansprüche, **dadurch gekennzeichnet, dass** der Dampf, welcher zwischen dem Deckel (9) und dem Kopfraum des Behälters eingeleitet wird, eine Überhitzung auf 120° bis 135° erfährt.

7. Verfahren nach einem beliebigen der Ansprüche 1 bis 6, **dadurch gekennzeichnet, dass** die Behälter bei einer Temperatur mit warmem, zuvor entgastem Produkt befüllt werden, die es mindestens ermöglicht, dass der Behälter bei Kontakt mit dem Produkt ohne weitere Hilfsmittel sterilisiert wird.

8. Verfahren nach Anspruch 1 und einem beliebigen der Ansprüche 2 bis 7, **dadurch gekennzeichnet, dass** die befüllte und verschlossenen Behälter eine Sterilisierung im Autoklaven erfahren, wobei der Druck während der Phasen der Temperaturerhöhung und -absenkung außerhalb des Behälter stets höher als derjenige im Inneren des Behälters ist.

9. Verfahren nach Anspruch 8, **dadurch gekennzeichnet, dass** der Gegendruck mindestens 300 mbar höher als der Druck im Behälter ist.

10. Verfahren nach den Ansprüchen 8 und 9, **dadurch gekennzeichnet, dass** der Druckunterschied zwischen dem Inneren des Behälters und der Autoklavenkammer 950 mbar nicht überschreitet.

11. Verfahren nach Anspruch 1, **dadurch gekennzeichnet, dass** das Produkt zuvor kontinuierlich sterilisiert und anschließend kontinuierlich abgekühlt wird, wobei das Produkt bei einer Temperatur eingedost wird, die niedriger als diejenige des pasteurisierten ist, zur Schonung der Zucker, und um eine Karamellisierung zu verhindern, sowie der Proteine oder der Farbe, und um die Maillard-Reaktion zu verhindern.

12. Anlage zur Durchführung des Verfahrens nach einem der vorhergehenden Ansprüche, wobei das kontinuierliche Vakuumverpacken von Lebensmitteln, die pasteurisiert und/oder sterilisiert werden können, in einem Behälter erfolgt, welcher mit einem Deckel verschlossen wird, der keinerlei Befestigungsmittel aufweist und mit einer elastischen Dichtung versehen ist, die durch ein Hochvakuum an Ort und Stelle gehalten wird, wobei die Anlage Folgendes aufweist:
- ein erstes Karussell (1) zur Bereitstellung von Behältern;
- ein zweites Karussell (2) zur Bereitstellung von Deckeln;
- ein drittes Karussell (3), das mit Gegenhaltern (8) versehen ist, die mit Hilfe von Magneten (10) die Deckel (9) vom Karussell (2) nehmen;
**gekennzeichnet durch**:
- einen Bereich (AA' und BB') des Karussell (3) zur Dampfeinleitung in den Raum, der zwischen dem Behälter und dem Deckel entsteht,
- einen Gegenhalter (8), der von einem Hohlkörper (5) getragen wird, auf welchem eine Druckfeder (7) sitzt sowie ein Ventil (6) zur Zuführung von Kühlwasser, wobei dieses sich **durch** Zusammendrücken der Feder öffnet, wobei das Kühlwasser sich auf den Deckel (9) ergießt und die Kondensation des Dampfes im Kopfraum sowie das angestrebte Vakuum hervorruft, indem der Behälter (11) mittels eines Kolbens in Richtung des Gegenhalters (8) gedrückt wird oder umgekehrt.

13. Anlage nach dem vorhergehenden Anspruch, **dadurch gekennzeichnet, dass** der Gegenhalter (8) mit einem Wasserkreislauf versehen ist, der mittels mindestens einer Eintrittsöffnung (12) und mindestens einer seitlichen Austrittsöffnung (13) durch den Hohlkörper (5) über den Deckel (9) verläuft.
